# EUROPÄISCHE PATENTSCHRIFT

(11) **EP 2 730 642 B1**
(45) Veröffentlichungstag und Bekanntmachung des Hinweises auf die Patenterteilung: **06.03.2019**
(21) Anmeldenummer: 13192223.9
(22) Anmeldetag: 08.11.2013
(51) Int. Cl.: C12M 1/107, C12M 1/00, E04H 7/06

(54) **Biogasbehälter**
Biogas container
Réservoir de biogaz

(30) Priorität: 09.11.2012 DE 202012104310 U
(43) Veröffentlichungstag der Anmeldung: 14.05.2014
(73) Patentinhaber: JOPE Beteiligungs GmbH, 87653 Eggenthal (DE)
(72) Erfinder: Baur, Peter, 87724 Ottobeuren (DE); Baur, Josef, 87784 Westerheim (DE)
(74) Vertreter: Pfister, Stefan Helmut Ulrich

(56) Entgegenhaltungen:
- EP-A1- 0 342 147
- EP-A2- 0 521 302
- AT-B- 388 158
- US-A1- 2003 066 789

## Beschreibung

Die Erfindung betrifft ein Biogasbehälter mit kegel- oder kuppelförmigem Tragluftdach nach dem Oberbegriff des unabhängigen Schutzanspruches 1.

Biogasbehälter werden innerhalb von Biogasanlagen eingesetzt, die zur Erzeugung von Biogas durch Vergärung von Biomasse dienen. In landwirtschaftlichen Biogasanlagen werden meist tierische Exkremente (Gülle, Festmist) und Energiepflanzen als Substrat eingesetzt. Bei den meisten Biogasanlagen wird das entstandene Gas vor Ort in einem Blockheizkraftwerk zur Strom- und Wärmeerzeugung genutzt.

Herkömmliche Biogasbehälter bestehen aus einem zylindrischen Grundkörper, der durch ein Dach, insbesondere Foliendach, abgedeckt ist, wobei das Foliendach entweder als einfache Siloabdeckung oder Doppelmembran-Gasspeicher mit Tragluftsystem ausgebildet ist.

Bei derartigen Biogasbehältern mit Tragluftdächern wird mit dem äußeren gasdichten und witterungsbeständigen Foliendach und einer darunter liegenden Folie ein Stützluftpolster aufgebaut. Mit Hilfe eines Gebläses erzeugt dieses Stützluftpolster einen konstanten Druck im Inneren des Biogasbehälters. So ergibt sich eine typische kegel- oder kuppelgewölbte Form des Tragluftdaches mit einer Druckdifferenz von 3 bis 5 Millibar. Unterhalb des Tragluftdaches kann eine tragende Konstruktion vorhanden sein, welche verhindert, dass die Gasspeicherfolie vom Gebläse nach unten gedrückt wird, oder die äußeren Witterungsbedingungen das Tragluftdach nach unten in den Biogasbehälter drücken.

Für einen optimalen Vergährungsprozeß wird das Substrat mit einer Heizung auf einem gewissen Temperaturniveau, zum Beispiel 38 - 40 °C gehalten.

Nachteile der gattungsgemäßen Biogasbehälter nach dem Stand der Technik sind, dass deren Dächer, ob nun einfache Abdeckungen, Doppelmembranabdeckungen oder Tragluftdächer stets zulassen, dass die im Behältermantel bestehende Wärme, nach oben hin, an den höchsten Punkt des Foliendaches abgeleitet wird. Hieraus resultiert ein schnelles Auskühlen des Substrates, das mit erhöhter Heizleistung zu kompensieren ist.

Dies wirkt sich negativ auf den Gesamtwirkungsgrad der gesamten Biogasanlage aus, so dass damit weniger Biostrom und Biowärme erzeugt werden kann.

Aus der AT 388 158 ist ein Gasspeicher bekannt, bei dem innerhalb einer Außenhülle zumindest eine flexible ballonartige Membrane aus flexiblen Flächengebilden vorgesehen ist, welche den Innenraum der Außenhülle in einen zwischen der Außenhülle und der Membran liegenden, mit einem Hilfsgas beaufschlagbaren Druckregelraum und zumindest einen innerhalb der Membran liegenden Gasspeicherraum unterteilt.

Aus der Druckschrift DE 31 11 745 A1 ist eine Fermentationsanlage zur Erzeugung von Gas aus Mist oder anderen organischen Stoffen bekannt. Diese weist einen Behälter für das organische Material auf, das fermentiert wird und ist auf der Oberseite durch ein Dach nach Art einer elastischen Membran abgedeckt.

Aus der Druckschrift US 2003/0066789 ist ein Abdecksystem für Klärbecken, Jauchegruben oder dergleichen bekannt. Diese Abdeckung wird flächig auf Auffangbehälter aufgelegt und mittels entsprechenden Stützen abgestützt. Für ein Tragluftdach beispielsweise für einen Biogasbehälter ist die in der US 2003/0066789 gezeigte Lösung nicht einsetzbar.

Aus der EP 0 521 302 ist eine Abdeckung von oben offenen Behältern bekannt, die insbesondere für Behälter, wie z.B. Güllegruben für die Biogas-Erzeugung vorgesehen ist. Hierbei sind wenigstens zwei übereinander angeordnete Folien vorgesehen, welche den Biogasbehälter rundherum am Grubenrand abdecken und die dort gasdicht befestigt sind. Dabei ist die erste Folie als Abdeckfolie bzw. als oben und außen liegende Folie kuppelartig konvex bezüglich des Behälters ausgebildet, während die zweite Folie konkav vorgesehen ist. Das erzeugte Biogas wird unterhalb der konkav ausgebildeten zweiten Folie abgesaugt.

Aufgabe der vorliegenden Erfindung ist es daher, den Stand der Technik derart weiterzubilden, dass der Wirkungsgrad einer Biogasanlage erhöht wird, bei gleichzeitiger Erhöhung der Lebensdauer des Tragluftdaches. Die gestellte Aufgabe wird durch die Merkmalskombination des unabhängigen Patentanspruches 1 gelöst. Wesentliches Merkmal hierbei ist, dass unterhalb der Innenfolie eine beabstandete, insbesondere kegelförmige oder kuppelförmige Wärmeschutzfolie vorgesehen ist, deren Material gasdicht ist und im Randbereich eine Anzahl von Biogasdurchtritts-Öffnungen aufweist. Die Wärmeschutzfolie ist dabei gasdicht bis auf die Vielzahl von Biogasdurchtritts-Öffnungen. Vorteil dieser Anzahl von Biogasdurchtritts-Öffnungen im Randbereich dieser erfindungsgemäßen Wärmeschutzfolie ist, dass nun die innerhalb des zylinderförmigen Behältermantels erzeugte beziehungsweise vorhandene Vergärungswärme nun nicht ungehindert nach oben hin in Richtung Aussenfolie oder Zwischenfolie aufsteigen kann, sondern gebremst und unterhalb der erfindungsgemäßen Wärmeschutzfolie dicht oberhalb des Flüssigkeitspegels der zu vergärenden Biomasse gesammelt wird und die Wärme damit dicht am Vergärungsprozess gehalten wird. Erst wenn sich ein beträchtliches Volumen an erwärmtem Biogas unterhalb der erfindungsgemäßen Wärmeschutzfolie angesammelt hat, kann ein kleiner Teil davon über die randseitig angeordneten zahlreichen Biogasdurchtritts-Öffnungen nach oben hin unter die Zwischenfolie oder aber die Aussenfolie (wenn die Zwischenfolie fehlt) hindurchtreten und dort aufgefangen werden. Die Ableitung des Biogases erfolgt dann von dort über entsprechende Ableitungen zu einem Auffangbehälter oder aber direkt in ein Blockheizkraftwerk oder dergleichen. Durch den erfindungsgemäßen Vorschlag wird das unerwünschte Auskühlen des Substrates erheblich verlangsamt. Der Energieaufwand zur Stabilisierung einer optimalen Substattemperatur sinkt.

Vorteilhafte Weiterbildungen der vorliegenden Erfindung sind Gegenstand der Unteransprüche.

Demzufolge zeichnet sich eine Weiterbildung des erfindungsgemäßen kegel- oder kuppelförmigen Tragluftdaches dadurch aus, dass sich zwischen Aussenfolie und Wärmeschutzfolie eine gasdichte Zwischenfolie befindet, die zur Aussenfolie und Wärmeschutzfolie beabstandet ist. Die Erfindung lässt sich allerdings, wie bereits erwähnt, auch für kegel- oder kuppelförmige Tragluftdächer einsetzen, die eine solche Zwischenfolie nicht aufweisen. Der Vorteil ist allerdings, dass die gasdichte Zwischenfolie in Verbindung mit der Wärmeschutzfolie einen Zwischenraum bildet, in dem das sich gebildete und gesammelte Biogas sicher aufgefangen kann und entsprechend auch über vorhandene Ableitungen dann in entsprechende Speicherbehälter oder aber direkt in ein Blockheizkraftwerk oder ein anderes Kraftwerk transportiert werden kann. Die Ableitungen des Gases kann dabei durch den eigenen Überdruck erfolgen, oder aber es wird ein Gebläse vorgesehen, das einen entsprechenden Absaugeffekt erzeugt, um das Biogas aus dem Zwischenraum zwischen Aussenfolie und Wärmeschutzfolie beziehungsweise zwischen der gasdichten Zwischenfolie und der Wärmeschutzfolie abzusaugen.

Im Rahmen der vorliegenden Erfindung soll die erfindungsgemäße Wärmeschutzfolie eingesetzt werden können für alle Arten von Biogasbehältern, welche mindestens eine Aussenfolie aufweisen, unabhängig davon, ob eine weitere Zwischenfolie vorhanden ist oder eben nicht.

Insbesondere eignet sich die erfindungsgemäße Wärmeschutzfolie jedoch für eine kegel- oder kuppelförmige Tragluftdachkonstruktion, bei der eine Zwischenfolie zwischen der Aussenfolie und der erfindungsgemäßen Wärmeschutzfolie vorgesehen ist.

Hierbei sind Aussenfolie, Zwischenfolie und Wärmeschutzfolie jeweils voneinander beabstandet übereinander oberhalb des zylindrischen Biogasmantels angeordnet, wobei die drei Folien sich lediglich im äußeren Randbereich des zylindrischen Biogasmantels berühren, da sie dort gasdicht mit dem Behältermantel verbunden sind, auf beliebige Art und Weise.

Eine vorteilhafte Weiterbildung der Erfindung zeichnet sich dadurch aus, dass der Kegel beziehungsweise die Kuppel der Wärmeschutzfolie sich konvex erstreckend auf dem Behältermantel angeordnet ist. Der Vorteil gegenüber den Lösungen des Standes der Technik ist der, dass dort entweder die Trennungen aus dem Reaktionsraum und dem Gassammelraum konkav, d.h. auf den Reaktionsraum zugewandt oder aber waagerecht vorgesehen sind. Nachteil einer solchen Ausgestaltung im Stand der Technik ist dadurch angegeben, dass das sich sammelnde Gas dann eben nicht, wie jetzt bei der erfindungsgemäßen Lösung, in der Kuppel beziehungsweise im Kegel in der Kegelspitze gesammelt wird, sondern vielmehr direkt nach außen in den Rand des Behälters transportiert wird und somit eine schnellere Abkühlung des Gases erfolgt. Bei der erfindungsgemäßen Lösung ist es jetzt gegeben, dass sich das gebildete Gas zunächst in der Kuppel sammelt und erst dann aus den Biogasdurchtritts-Öffnungen austritt, wenn sich genügend Gas in der Kuppel gesammelt hat. Dadurch bleibt auch die Reaktionswärme in der Reaktionskammer erhalten und es ist nicht notwendig, zusätzlich zu heizen oder zumindest nicht notwendig, so viel zu heizen, wie dies im Stand der Technik bisher notwendig war. Demzufolge ist die Ausgestaltung nach der Erfindung mit einer konvex ausgebildeten Wärmeschutzfolie, d.h., die Wärmeschutzfolie erstreckt sich in Einbaustellung nach oben und nicht nach unten, wie im Stand der Technik, besser als die Lösungen des Standes der Technik, weil eben der Energieeinsatz insgesamt um die Reaktionstemperatur zu erhalten deutlich verringert wird. Die eigene Reaktionswärme insbesondere auch des gebildeten Gases wird jetzt besser im Reaktionsbereich gehalten, was sich insgesamt vorteilhaft auf die Energiebilanz der Biogasanlage auswirkt.

Zur besseren Wärmeisolation ist die erfindungsgemäße Wärmeschutzfolie relativ dick und damit schwer ausgebildet, und wird erfindungsgemäß auf einer Tragkonstruktion abgestützt, welche insbesondere als Tragpfosten ausgebildet ist, der sich in der Mitte des zylindrischen Behältermantels befindet und auf dem Boden beziehungsweise dem Fundament des Mantels beziehungsweise Biogasbehälters abstützt. Natürlich kann zusätzlich zu diesem Tragpfosten eine weitere dachartige Tragkonstruktion vorhanden sein, die sich dann am oberen Rand des Zylindermantels abstützt. Diese dachartige Tragkonstruktion kann auch separat vorgesehen sein, ohne diesen zuvor genannten vertikalen Stützpfosten.

Als Material aller Folien, nämlich der Aussenfolie, der Zwischenfolie und der Wärmeschutzfolie werden bevorzugt Kunststoffmaterialien gewählt, insbesondere glasfaserverstärkter Kunststoff (GFK) oder aber kohlefaserverstärkter Kunststoff (KFK).

Von besonderem Vorteil ist es nach der Erfindung entsprechend einer bevorzugten Weiterbildung, wenn die Wärmeschutzfolie mehrschichtig ausgebildet ist. Dabei ist zumindest eine Schicht aus die Wärme schlecht leitendem Material gebildet. Es ist weiterhin nach der Erfindung durchaus vorgesehen, dass die Wärmeschutzfolie mit Luft oder Gas gefüllten, vorzugsweise noppenförmig ausgebildeten Zwischenräumen versehen ist. Dadurch wird natürlich die Wärmedämmung insgesamt noch erhöht, was eine weitere Effizienzerhöhung hinsichtlich der Energiebilanz bewirkt. Auch können wärmedämmende Materialien in den Zwischenräumen angeordnet sein.

Von Vorteil ist es nach der Erfindung auch, wenn, wie in einer Variante vorgeschlagen, die Wärmeschutzfolie auf der dem Substrat zugewandten Seite (in Einbaustellung nach unten) eine wärmeabstrahlende Schicht vorgesehen ist. Diese wärmeabstrahlende Schicht führt dazu, dass die nach oben aufsteigende Wärme teilweise wieder reflektiert wird und in den Reaktionsraum aufgrund von Wärmestrahlung zurückgeführt wird, sodass ein weiterer positiver Effekt hinsichtlich der einzusetzenden Energie ensteht, was die Gesamtbilanz verbessert beziehungsweise den gesamten Wirkungsgrad der Biogasanlage weiter erheblich erhöht.

Was nun die Ausführung der randseitig an der erfindungsgemäßen Wärmeschutzfolie angeordneten Biogasdurchtritts-Öffnungen anbelangt, so könnten diese im Rahmen der vorliegenden Erfindung vollkommen frei gewählt werden, insbesondere sind diese aber kreisrund, oval, quadratisch, rechteckig oder aber auch polygonal, oder aber sind auf einem Umkreis um den zentralen Pol der Wärmeschutzfolie als eine Vielzahl von Langlöchern ausgebildet.

Auch können natürlich im Rahmen der vorliegenden Erfindung mehrere Reihen von Biogasdurchtritts-Öffnungen auf unterschiedlichen Umkreisen um den Pol der Wärmschutzfolie vorgesehen sein, wobei dann die Biogasdurchtritts-Öffnungen benachbarter Reihen umfänglich zueinander versetzt angeordnet sind.

Von besonderem Vorteil ist es, wenn wie in einer Weiterbildung der Erfindung vorgeschlagen, die Biogasdurchtritts-Öffnungen mittels einer Abdeckung verschließbar und öffenbar sind. Dabei ist es vorgesehen, dass die Abdeckung auf einer von einem Substrat, welches im Behälter angeordnet oder anordenbar ist, abgewandten Seite beziehungsweise in Einbaustellung auf der Oberseite der Wärmeschutzfolie angeordnet ist. Diese Abdeckungen bewirken, dass der Druckaufbau in der Kuppel beziehungsweise im Kegel insgesamt noch günstiger erfolgt und sich diese Abdeckungen erst dann öffnen, wenn ausreichend Biogas erzeugt wurde. Dadurch wird natürlich die Wärmeabstrahlung nach unten hin zum Substrat beziehungsweise zum Reaktionsgemisch noch günstiger. Die Wärme wird insgesamt länger im Biogasbehälter, also im Reaktionsraum, gehalten und es weicht nur dann in den Zwischenraum zwischen der Aussenfolie und der Wärmeschutzabdeckung aus, wenn sich ein genügender Druckaufbau gebildet hat.

Aus diesem Grund ist es natürlich von weiterem Vorteil, wenn die Abdeckungen der Biogasdurchtritts-Öffnungen klappenförmig ausgebildet ist. Klappenförmig bedeutet, dass natürlich die Abdeckung sich der Form der Biogasdurchtritts-Öffnung anpasst.

Allerdings derart, dass sie zumindest einseitig festgelegt ist, sodass die Abdeckung sich eben erst dann öffnet, wenn sich ausreichend Biogas im Reaktionsraum befindet. Von besonderem Vorteil ist es dabei, wenn die Abdeckung sich bei einem vorwählbaren und/oder einstellbaren Überdruck öffnet und bei nachlassendem Druck im Behälter wieder schließt. Dies kann in einfacher Weise dadurch geschehen, dass ein entsprechendes Gewicht der Abdeckung, d.h. eine entsprechend stark oder dick gewählte Folie vorgesehen wird, für die ein bestimmter Druck notwendig ist, um das Gewicht anzuheben. Dies geschieht dann automatisch bei entsprechendem Überdruck. Selbstverständlich kann man dies technisch auch so lösen, dass man die Abdeckung vom Gewicht her so auswählt, dass ein bestimmter Überdruck im Reaktionsbereich entstehen muss, um diese Abdeckung abzuheben.

Weiterhin wird im Rahmen einer bevorzugten Weiterbildung dieser Erfindung vorgesehen, dass die Ränder der Biogasdurchtritts-Öffnungen verstärkt sind, zum Beispiel durch eine Verkettelung der Ränder mit Garn, oder aber durch das Einbringen von Durchgangsnieten aus Kunststoff oder aus Leichtmetall, oder aber durch das Aufschweissen eines Verstärkungsringes aus dem gleichen Material wie die Wärmeschutzfolie selbst.

In der Zeichnung ist die Erfindung insbesondere in einem Ausführungsbeispiel schematisch dargestellt. Es zeigen:
- Fig. 1:: eine Seitenansicht im Schnitt auf den erfindungsgemäßen Biogasbehälter mit kuppelförmigem Tragluftdach;
- Fig. 2:: eine Draufsicht auf Fig. 1 mit abgenommener Außen- und Zwischenfolie;
- Fig. 3:: einen vergrößerten Ausschnitt der Biogasdurchtritts-Öffnung aus Fig. 1.

In den Figuren sind gleiche oder einander entsprechende Elemente jeweils mit den gleichen Bezugszeichen bezeichnet und werden daher, sofern nicht zweckmäßig, nicht erneut beschrieben.

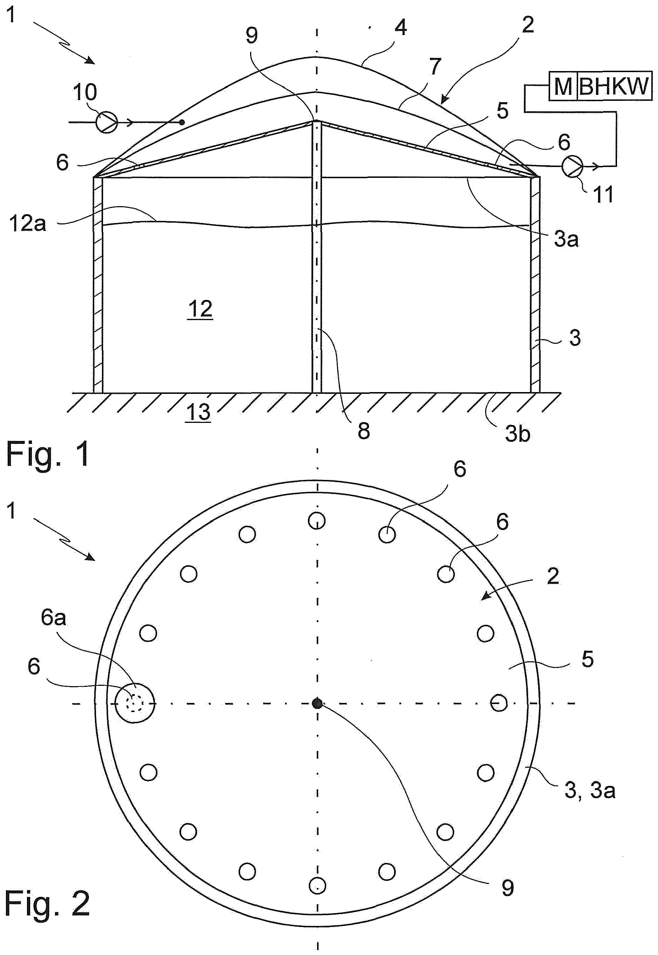

Fig. 1 zeigt einen Längsschnitt durch die Hochachse eines erfindungsgemäßen Biogasbehälters 1, der aus einem Tragluftdach 2 besteht, welches auf der Oberseite 3a eines Behältermantels 3 aufgelegt ist und dort sich abstützt und festgelegt ist.

Die Dachkonstruktion des Tragluftdaches 2 besteht in diesem Beispiel aus insgesamt drei Folien, nämlich aus einer äußeren Aussenfolie 4, welche den Wetter- und Windschutz darstellt und eine sehr stabile Folie sein muss. Darunter befindet sich in diesem Beispiel eine Zwischenfolie 7, welche den Biogasdruck regelt, aber in anderen Ausführungsformen jedoch auch vollkommen entfallen kann. Unter diesen beiden Folien 4, 7 beziehungsweise unter der Aussenfolie 4 befindet sich die erfindungsgemäße Wärmeschutzfolie 5, die mit ihrem Randbereich auf der Oberseite 3a des Behältermantels 3 aufgelegt und dort befestigt ist und die im Bereich ihres Poles 9 sich auf einem vertikalen Tragpfosten 8 abstützt, welcher mit seinem anderen Ende auf dem Boden des Behälters 3 abgestützt ist beziehungsweise auf dem Fundament 13 des gesamten Biogasbehälters 1.

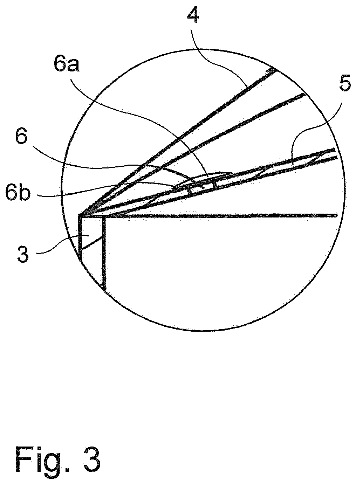

Wie insbesondere in der Zusammenschau der Fig. 1 mit der Fig. 2 zu erkennen ist, sind im Randbereich der erfindungsgemäßen Wärmeschutzfolie 5 eine Vielzahl von Biogasdurchtritts-Öffnungen 6 vorgesehen auf einem gemeinsamen Umkreis um den Bereich des Poles 9 der Wärmeschutzfolie 5 herum. Beispielhaft sind diese Biogasdurchtritts-Öffnungen 6 als kreiszylindrische Öffnungen ausgeführt, können jedoch in anderen Ausführungsformen eine beliebige Form annehmen und sollten so gewählt werden, dass die Reissfestigkeit der Wärmeschutzfolie immer noch den gestellten Anforderungen genügt. Die Biogasdurchtritts-Öffnungen 6 sind vorzugsweise mit einer Abdeckung 6a abgedeckt. Diese Ausführungsform ist als vergrößerter Ausschnitt in Fig. 3 gezeigt. Die Abdeckung 6a ist dabei klappenförmig ausgebildet, weshalb sie einseitig über einen bestimmten Abschnitt der Abdeckung 6a flexibel an einem Befestigungspunkt beziehungsweise -abschnitt 6b festgelegt ist.

In Fig. 1 ist mit der Positionsnummer 10 ein Luftgebläse markiert, welches Druckluft von der Umgebung her in den Zwischenraum zwischen der Aussenfolie 4 und der Zwischenfolie 7 einbringt und somit die Aussenfolie 4 stabil in Kuppelform bringt. Wie bereits zuvor beschrieben, kann jedoch die Zwischenfolie 7 vollkommen entfallen, so dass die Druckluft über das Gebläse 10 in den Zwischenraum zwischen der Aussenfolie 4 und der Wärmeschutzfolie 5 eingebracht wird.

Mit der Positionsnummer 11 in Fig. 1 ist ein Absauggebläse dargestellt, welches das durch den Gärungsprozess erzeugte Biogas aus dem Zwischenraum zwischen der Wärmeschutzfolie 5 und der gasdichten Zwischenfolie 7 bzw. der Wärmeschutzfolie 5 und der Aussenfolie 4 absaugt und einem Verbrennungsmotor beziehungsweise einem Blockheizkraftwerk zuführt, zur Erzeugung von Strom und/oder Wärme.

## Patentansprüche

1. Biogasbehälter mit kegel- oder kuppelförmigem Tragluftdach, wobei das Tragluftdach eine gasdichte und gegebenenfalls auch flüssigkeitsdichte Aussenfolie beinhaltet, die auf der offenen Oberseite eines Behältermantels mit insbesondere kreisrunder oder eckiger Grundform aufbringbar ist, wobei unterhalb der Aussenfolie (4) eine beabstandete Wärmeschutzfolie (5) vorgesehen ist, **dadurch gekennzeichnet, dass** die Wärmeschutzfolie (5), welche insbesondere kegel- oder kuppelförmig ist, gasdicht ist bis auf eine Vielzahl von Biogasdurchtritts-Öffnungen (6) in ihrem Randbereich, und wobei sie sich auf einem Tragpfosten (8) und/oder Traggerüst abstützt.

2. Biogasbehälter nach Anspruch 1, **dadurch gekennzeichnet, dass** sich zwischen Aussenfolie (4) und Wärmeschutzfolie (5) eine gasdichte Zwischenfolie (7) befindet, die zur Aussenfolie (4) und Wärmeschutzfolie (5) beabstandet ist.

3. Biogasbehälter nach Anspruch 1, **dadurch gekennzeichnet, dass** der Kegel bzw. die Kuppel der Wärmeschutzfolie (5) sich konvex erstreckend auf dem Behältermantel (3) angeordnet ist.

4. Biogasbehälter nach einem der Ansprüche 1 bis 3, **dadurch gekennzeichnet, dass** die Aussenfolie (4) und die Wärmeschutzfolie (5) und die Zwischenfolie (7) aus Kunststoff insbesondere aus glasfaserverstärktem Kunststoff (GFK), kohlefaserverstärktem Kunststoff (KFK), Polyethylen (PE), Polypropylen (PP), Polyvinylchlorid (PVC), Polystyrol (PS), Polyurethan (PU), Polyethylenterephtalat (PET), Polytertafluorethylen (PTFE) oder Polymethylmethacrylat (PMMA) bestehen.

5. Biogasbehälter nach Anspruch 1, **dadurch gekennzeichnet, dass** die Wärmeschutzfolie (5) mehrschichtig ausgebildet ist, wobei zumindest eine Schicht aus die Wärme schlecht leitendem Material gebildet ist und/oder die Wärmeschutzfolie (5) mit Luft oder Gas gefüllte, vorzugsweise noppenförmig ausgebildete Zwischenräume aufweist.

6. Biogasbehälter nach Anspruch 1, **dadurch gekennzeichnet, dass** auf der dem Substrat zugewandten Seite der Wärmeschutzfolie (5) eine Wärme abstrahlende Schicht vorgesehen ist.

7. Biogasbehälter nach einem der Ansprüche 1 bis 4, **dadurch gekennzeichnet, dass** die Biogasdurchtritts-Öffnungen (6) über die Randfläche der Wärmeschutzfolie (5) gleichmäßig verteilt sind, z.B. zwischen 1-10 cm, bzw. 1-10°, dass der Abstand der Biogasdurchtritts-Öffnungen (6) zum Rand der Wärmeschutzfolie zwischen 10 bis 50 cm liegt und dass die Biogasdurchtritts-Öffnungen (6) eine Fläche zwischen 10 bis 100 cm2 aufweisen.

8. Biogasbehälter nach einem der Ansprüche 1 bis 5, **dadurch gekennzeichnet, dass** die Biogasdurchtritts-Öffnungen (6) kreisrund, oval, quadratisch, rechteckig oder polygonal oder als Langloch auf einem Umkreis um den zentralen Pol (9) der Wärmeschutzfolie (5) ausgebildet sind.

9. Biogasbehälter nach einem der Ansprüche 1 bis 6, **dadurch gekennzeichnet, dass** mehrere Reihen von Biogasdurchtritts-Öffnungen (6) auf unterschiedlichen Umkreisen um den Pol (9) der Wärmeschutzfolie (5) vorgesehen sind und die Biogasdurchtritts-Öffnungen (6) benachbarter Reihen umfänglich zueinander versetzt angeordnet sind.

10. Biogasbehälter nach Anspruch 1, **dadurch gekennzeichnet, dass** die Biogasdurchtritts-Öffnungen (6) vorzugsweise mittels einer Abdeckung (6a) verschließbar und öffenbar sind, wobei die Abdeckung (6a) insbesondere auf einer von einem Substrat, welches im Behälter (3) angeordnet oder anordenbar ist abgewandten Seite bzw. in Einbaustellung auf der Oberseite der Wärmeschutzfolie (5) angeordnet ist.

11. Biogasbehälter nach Anspruch 10, **dadurch gekennzeichnet, dass** die Abdeckung (6a) der Biogasdurchtritts-Öffnung (6) klappenförmig ausgebildet ist, wobei die Abdeckung (6a) sich bei einem vorwählbaren und/oder einstellbaren Überdruck öffnet und bei nachlassendem Druck im Behälter wieder schließt.

12. Biogasbehälter nach einem der vorhergehenden Ansprüche, **dadurch gekennzeichnet, dass** die Ränder der Biogasdurchtritts-Öffnungen (6) verstärkt sind, z.B. durch eine Verkettelung mit Garn, oder durch Einbringen von Durchgangsnieten aus Kunststoff oder Leichtmetall, oder durch Aufschweissen eines Verstärkungsringes aus gleichem Kunststoffmaterial wie die Wärmeschutzfolie (5).

## Claims

1. Biogas container with a cone- or dome-shaped air supported roof, wherein the air supported roof contains a gas-tight and, if necessary, also liquid-tight exterior film that can in particular be applied on the open upper side of a container shell with in particular circular or angular basic shape, wherein below the exterior film (4) a spaced insulating film (5) is provided, **characterized in that** the insulating film (5), that is in particular cone- or dome-shaped, is gas-tight with the exception of a multitude of biogas passage openings (6) in its peripheral area, and wherein it is supported on a support post (8) and/or support frame.

2. The biogas container according to claim 1, **characterized in that** between exterior film (4) and insulating film (5) a gas-tight intermediate film (7) is located that is spaced towards the exterior film (4) and insulating film (5).

3. The biogas container according to claim 1, **characterized in that** the cone or the dome of the insulating film (5) is arranged extending convexly on the container shell (3).

4. The biogas container according to any one of the claims 1 to 3, **characterized in that** the exterior film (4) and the insulating film (5) and the intermediate film (7) consist of plastic material, in particular glass fiber reinforced plastic material (GRP), carbon fiber reinforced plastic material (CRP), polyethylene (PE), polypropylene (PP), polyvinyl chloride (PVC), poly styrene (PS), poly urethane (PU), polyethylene terephtalate (PET), poly tetra fluorine ethylene (PTFE) or polymethyl methacrylate (PMMA).

5. The biogas container according to claim 1, **characterized in that** the insulating film (5) consists of several layers, wherein at least one layer is formed by poorly heat conducting material, and/or the insulating film (5) has preferably knoblike spaces that are filled with air or gas.

6. The biogas container according to claim 1, **characterized in that** on the side of the insulating film (5) facing the substrate a layer that radiates heat is provided.

7. The biogas container according to any one of the claims 1 to 4, **characterized in that** the biogas passage openings (6) are spread evenly across the peripheral area of the insulating film (5), for example between 1 and 10 cm, or 1 and 10°, so that the distance between the biogas passage openings (6) and the edge of the insulating film is between 10 and 50 cm, and that the biogas passage openings (6) have a surface between 10 and 100 cm².

8. The biogas container according to any one of the claims 1 to 5, **characterized in that** the biogas passage openings (6) are formed circularly, ovally, squarely, rectangularly, or polygonally, or as longitudinal hole on a circumference around the central pole (9) of the insulating film (5).

9. The biogas container according to any one of the claims 1 to 6, **characterized in that** several rows of biogas passage openings (6) are provided on different circumferences around the pole (9) of the insulating film (5), and the biogas passage openings (6) of adjacent rows are arranged staggered on the circumference.

10. The biogas container according to claim 1, **characterized in that** the biogas passage openings (6) can be opened and closed preferably by means of a cover (6a), wherein the cover (6a) is arranged in particular on a side opposite of a substrate, that is or can be arranged in the container (3), or is arranged in installation position on the upper side of the insulating film (5) .

11. The biogas container according to claim 10, **characterized in that** the cover (6a) of the biogas passage opening (6) is formed like a flap, wherein the cover (6a) opens, when a preselected and/or set overpressure is reached, and closes when the pressure in the container recedes.

12. The biogas container according to any one of the preceding claims, **characterized in that** the edges of the biogas passage openings (6) are reinforced, for example by interlinking with yarn, or applying passage rivets of plastic or light metal, or welding a reinforcement ring of the same plastic material as the insulating film (5).

## Revendications

1. Réservoir de biogaz possédant un plafond à membrane en forme de cône ou de coupole construite de façon à ce que le plafond comporte une membrane extérieure étanche aux gaz et le cas échéant étanche aux liquides pouvant être montée sur la face ouverte d'une parois de réservoir d'une forme de base en particulier circulaire ou polygonale de façon à ce qu'une couche d'isolation thermique (5) est prévue à une certaine distance en dessous de la membrane extérieure (4), **caractérisé en ce que** la membrane d'isolation thermique (5) en particulier en forme de cône ou de coupole est étanche aux gaz à l'exception de plusieurs ouvertures de passage de biogaz (6) situées dans la zone du bord de la membrane et **en ce qu'**elle s'appuie sur un poteau de support (8) et/ou sur une armature de support.

2. Réservoir de biogaz selon la revendication 1, **caractérisé en ce qu'**une membrane intermédiaire (7) étanche aux gaz est prévue entre la membrane extérieure (4) et la membrane d'isolation thermique (5) et à une certaine distance par rapport à cette membrane extérieure (4) et la membrane d'isolation thermique (5).

3. Réservoir de biogaz selon la revendication 1, **caractérisé en ce que** le dôme conique ou la coupole formée par la membrane d'isolation thermique (5) se tend selon une forme convexe sur la paroi du réservoir (3).

4. Réservoir de biogaz selon une des revendications 1 à 3, **caractérisé en ce que** la membrane extérieure (4), la membrane d'isolation thermique (5) et la membrane intermédiaire (7) sont fabriquées en plastique et en particulier en plastique renforcé de fibres de verre (GFK), en plastique renforcé de fibres de carbone (KFK), en polyéthylène (PE), en polypropylène (PP), en polychlorure de vinyle (PVC), en polystyrène (PS), en polyuréthane (PU), en polytéréphtalate d'éthylène (PET), en polytétrafluoroethylène (PTFE) ou en polyméthacrylate de méthyle (PMMA).

5. Réservoir de biogaz selon la revendication 1, **caractérisé en ce que** la membrane d'isolation thermique (5) est réalisée en multicouche dont au moins une couche consiste en un matériau conduisant mal la chaleur et/ou **en ce que** la membrane d'isolation thermique (5) comporte des alvéoles remplies d'air ou de gaz et de préférence en forme de bouton.

6. Réservoir de biogaz selon la revendication 1, **caractérisé en ce qu'**une couche renvoyant la chaleur est prévue sur la surface de la membrane d'isolation thermique faisant face au substrat.

7. Réservoir de biogaz selon une des revendications 1 à 4, **caractérisé en ce que** les ouvertures de passage du biogaz (6) sont distribuées uniformément sur la zone du bord de la membrane d'isolation thermique (5), c'est-à-dire entre 1 -10cm ou 1 - 10° afin d'obtenir une distance entre les ouvertures de passage du biogaz (6) vers le bord de la membrane d'isolation thermique allant de 10 à 50cm avec une surface couverte par les ouvertures de passage du biogaz entre 10 à 100cm².

8. Réservoir de biogaz selon une des revendication 1 à 5, **caractérisé en ce que** les ouvertures de passage du biogaz (6) ont une forme circulaire, ovale, carrée, rectangulaire ou polygonale ou oblongue et **en ce qu'**elles sont situées sur une circonférence autour du pôle central (9) de la membrane d'isolation thermique (5).

9. Réservoir de biogaz selon une des revendications 1 à 6, **caractérisé en ce que** plusieurs lignes d'ouvertures de passage du biogaz (6) sont prévues sur des circonférences différentes autour du pôle de la membrane d'isolation thermique (5) et **en ce que** les ouvertures de passage de biogaz (6) de lignes voisines sont décalées sur leurs circonférences respectives.

10. Réservoir de biogaz selon la revendication 1, **caractérisé en ce que** les ouvertures de passage du biogaz (6) peuvent de préférence être fermées ou ouvertes à l'aide d'un couvercle (6a) lequel est en particulier situé sur la face opposée au substrat du réservoir (3) ou pouvant être mis dans le réservoir ou en état monté sur la face supérieure de la membrane d'isolation thermique (5).

11. Réservoir de biogaz selon la revendication 10, **caractérisé en ce que** le couvercle (6a) de l'ouverture de passage du biogaz (6) est réalisé sous la forme d'un clapet de façon à ce que le couvercle (6a) s'ouvre sous l'effet d'une surpression prédéfinie et/ou ajustable et se ferme lorsque que la pression dans le réservoir diminue.

12. Réservoir de biogaz selon une des revendication précédentes, **caractérisé en ce que** les bords des ouvertures de passage de biogaz (6) sont renforcés par exemple par chainage avec du fil ou par l'introduction de rivets oeillet en plastique ou en métal léger ou par fixation par soudage d'un anneau de renfort du même matériau plastique que la membrane d'isolation thermique (5).
